# EUROPÄISCHE PATENTSCHRIFT

(11) **EP 2 209 823 B1**
(45) Veröffentlichungstag und Bekanntmachung des Hinweises auf die Patenterteilung: **06.07.2011**
(21) Anmeldenummer: 08848864.8
(22) Anmeldetag: 13.11.2008
(51) Int. Cl.: C08F 220/06, C08F 220/18, C08F 222/06, C08F 265/02, C08F 265/06, C08F 267/02, C11D 3/00, C08F 2/22

(54) **VERFAHREN ZUR HERSTELLUNG EINER VERDICKER-DISPERSION**
METHOD FOR PRODUCING A THICKENER DISPERSION
PROCÉDÉ DE FABRICATION D'UNE DISPERSION D'ÉPAISSISSANT

(30) Priorität: 14.11.2007 EP 07120725
(43) Veröffentlichungstag der Anmeldung: 28.07.2010
(73) Patentinhaber: BASF SE, 67056 Ludwigshafen (DE)
(72) Erfinder: LEYRER, Reinhold J., 67125 Dannstadt-Schauernheim (DE); SCHMIDT, Kati, 67063 Ludwigshafen (DE)
(86) Internationale Anmeldenummer: PCT/EP2008/065447
(87) Internationale Veröffentlichungsnummer: WO 2009/062994

(56) Entgegenhaltungen:
- EP-A- 0 013 836
- EP-A- 0 658 579
- EP-A- 0 729 989
- US-A- 4 423 199
- US-A- 4 569 965

## Beschreibung

Die Erfindung betrifft ein Verfahren zur Herstellung einer Verdicker-Dispersion, d. h. einer wässrigen Dispersion eines alkalilöslichen Copolymers, die sich als Verdicker eignet. Die Erfindung betrifft außerdem die so erhältliche Verdicker-Dispersion und ihre Verwendung zur Rheologiemodifizierung von Papier-Streichmassen, Textildruckpasten, Arzneimitteln, kometischen Mitteln, Waschmitteln, Reinigungsmitteln oder Nahrungsmitteln.

"Alkalilöslich" bedeutet, dass die Zugabe von Alkali zur Dispersion zur Auflösung oder Quellung der dispergierten Copolymerpartikel und zu einer Verdickung und Viskositätszunahme führt. Um diesen Verdickungseffekt zu erhalten, enthalten die Copolymere einpolymerisierte Einheiten ethylenisch ungesättigter Carbonsäuren neben Einheiten hydrophober Monomere.

Eine besondere Form der alkalilöslichen Verdicker sind Assoziativverdicker. Die Assoziativverdicker verfügen über hydrophobe oder tensidartige Seitengruppen. Sie sind in der Lage, in einem hydrophilen Medium sowohl mit sich selbst als auch mit anderen hydrophoben Stoffen, wie Tensidmicellen, Pigmenten, Bindemittelteilchen oder Füllstoffen, in Wechselwirkung zu treten und nichtkovalente Netzwerke zu bilden. Durch das daraus resultierende assoziative Netzwerk wird das Medium verdickt oder geliert.

Die EP-A 0 013 836 offenbart Emulsionscopolymere, die (i) 20 bis 69,5 Gew.-% (Meth)acrylsäure, (ii) 0,5 bis 25 Gew.-% eines Monomers der Formel CH₂=C(R)-C(O)-O-(CH₂CH₂O)ₙ-R⁰, worin R für H oder CH₃ steht, n wenigstens 2 ist und R⁰ für C₈-C₃₀-Alkyl steht, und (iii) wenigstens 30 Gew.-% eines C₁-C₄-Alkyl(meth)acrylats enthalten. Nach Neutralisation mit Alkali dienen die Copolymere als Verdicker für Anstrichmittel, Waschmittel und dergleichen.

Die WO 99/65958 beschreibt alkalilösliche Verdicker, die das Umsetzungsprodukt einer ungesättigten Carbonsäure, eines monoethylenisch ungesättigten Monomers und eines hydrophoben, alkoxylierten Macromonomers umfassen. Das monoethylenisch ungesättigte Monomer umfasst eine Methylgruppe; vorzugsweise handelt es sich um Methylacrylat. Diese Polymere sollen bereits bei pH 4,5 bis 6,0 löslich werden und sich daher für kosmetische Erzeugnisse eignen.

Die WO 2006/016035 betrifft die Verwendung eines wasserlöslichen Acrylpolymers als Verdickungsmittel in pigmentierten wässrigen Zubereitungen. Das Acrylpolymer besteht aus einem ethylenisch ungesättigten Monomer mit Carboxylfunktion, einem ethylenisch ungesättigten nichtionischen Monomer und einem ethylenisch ungesättigten oxyalkylierten Monomer, das mit einer hydrophoben nicht-aromatischen verzweigten Kette mit 10 bis 24 Kohlenstoffatomen terminiert ist.

Typischerweise werden die Verdicker-Dispersionen durch eine chargenweise Polymerisation (Batch-Polymerisation) hergestellt. Die chargenweise Polymerisation weist den Vorzug auf, dass sie hohe Umsätze und hohe Molekulargewichte der Copolymere liefert, die für eine optimale Verdickungswirkung erwünscht sind. Der Nachteil der chargenweisen Polymerisation liegt in einer schwierigen Steuerbarkeit des Polymerisationsverlaufs, die zu ungenügender Batch-to-Batch-Reproduzierbarkeit führen kann. Daneben birgt die chargenweise Polymerisation im industriellen Maßstab auch Sicherheitsprobleme, da eine große Menge polymerisationsfähiger Verbindungen, wie sie bei der Batch-Polymerisation vorgelegt wird, bei unvorhergesehenem Verlauf der Polymerisation zu explosionsartigen Reaktionen führen kann.

Der Erfindung liegt die Aufgabe zugrunde, die vorstehend geschilderten Probleme zu beheben.

Die Aufgabe wird erfindungsgemäß gelöst durch ein Verfahren zur Herstellung einer wässrigen Verdicker-Dispersion aus einer Monomerzusammensetzung, die umfasst:
a) wenigstens eine ethylenisch ungesättigten Carbonsäure, und
b) wenigstens ein ethylenisch ungesättigtes hydrophobes Monomer,
wobei man
(i) aus 10 bis 80 Gew.-%, vorzugsweise 40 bis 70 Gew.-%, der Monomerzusammensetzung eine zumindest teilweise polymerisierte Voremulsion herstellt und
(ii) die Restmenge der Monomerzusammensetzung vollständig zu der zumindest teilweise polymerisierten Voremulsion zugibt und mittels eines Redoxinitiator systems eine radikalische Polymerisation initiiert.

Üblicherweise stellt man im Schritt (i) die zumindest teilweise polymerisierte Voremulsion in Gegenwart eines thermisch aktivierbaren Initiators oder eines Redoxinitiators her. Die Verwendung eines thermisch aktivierbaren Initiators ist bevorzugt.

Geeignete thermisch aktivierbare radikalische Initiatoren sind vor allem solche des Peroxy- und Azotyps. Hierzu zählen unter anderem Wasserstoffperoxid, Peressigsäure, t-Butylhydroperoxid, Di-t-butylperoxid, Dibenzoylperoxid, Benzoylhydroperoxid, 2,4-Dichlorbenzoylperoxid, 2,5-Dimethyl-2,5-bis(hydroperoxy)hexan, Perbenzoesäure, t-Butylperoxypivalat, t-Butylperacetat, Dilauroyl peroxid, Dicapryloylperoxid, Distearoylperoxid, Dibenzoylperoxid, Diisopropylperoxydicarbonat, Didecylperoxydicarbonat, Dieicosylperoxydicarbonat, Di-t-butylperbenzoate, Azobisisobutyronitril, 2,2'-Azobis-2,4-dimethylvaleronitril, Ammoniumpersulfat, Kaliumpersulfat, Natriumpersulfat und Natriumperphosphat.

Zweckmäßig ist der Einsatz von thermisch aktivierbaren radikalischen Initiatoren, deren im Schritt (i) nicht umgesetzte Anteile als Oxidationsmittelkomponente eines Redoxinitiatorsystems für die Initiierung der Polymerisation im Schritt (ii) dienen können. Die Persulfate (Peroxodisulfate), insbesondere Natriumpersulfat, sind am meisten bevorzugt.

Bei der Durchführung der Emulsionspolymerisation wird der Initiator in ausreichender Menge verwendet, um die Polymerisationsreaktion zu initiieren. Der Initiator wird üblicherweise in einer Menge von etwa 0,01 bis 3 Gew.-%, bezogen auf das Gesamtgewicht der eingesetzten Monomere, verwendet. Die Initiatormenge beträgt vorzugsweise etwa 0,05 bis 2 Gew.-% und insbesondere 0,1 bis 1 Gew.-%, bezogen auf das Gesamtgewicht der eingesetzten Monomere.

Die Emulsionspolymerisation erfolgt üblicherweise bei 35 bis 140 °C. Sie kann sowohl als Batch-Prozess als auch in Form eines Zulaufverfahrens durchgeführt werden. Bevorzugt ist die Monomer-Zulauffahrweise, bei dem man zumindest einen Teil des Polymerisationsinitiators und gegebenenfalls einen Teil der Monomeren vorlegt, auf Polymerisationstemperatur erhitzt und anschließend den Rest des Polymerisationsansatzes, üblicherweise über mehrere getrennte Zuläufe, von denen einer oder mehrere die Monomeren in reiner oder emulgierter Form enthalten, kontinuierlich oder stufenweise unter Aufrechterhaltung der Polymerisation zuführt. Vorzugsweise erfolgt der Monomerzulauf in Form einer Monomeremulsion. Parallel zum Monomerzulauf kann weiterer Polymerisationsinitiator zudosiert werden.

In bevorzugten Ausführungsformen legt man im Schritt (i) die gesamte Initatormenge vor, d. h. parallel zum Monomerzulauf erfolgt keine weitere Initiatordosierung. Es wurde überraschend gefunden, dass diese Vorgehensweise zu besonders hoher Transparenz und Verdickungsleistung des Verdickers führt.

In einer bevorzugten Ausführungsform legt man daher den thermisch aktivierbaren radikalischen Polymerisationsinitiator vollständig vor und lässt das Monomerengemisch, vorzugsweise in Form einer Monomeremulsion, zulaufen. Bevor man den Zulauf des Monomerengemisches startet, bringt man die Vorlage auf die Aktivierungstemperatur des thermisch aktivierbaren radikalischen Polymerisationsinitiators oder eine höhere Temperatur. Als Aktivierungstemperatur wird die Temperatur angesehen, bei der die Hälfte des Initiators nach zehn Stunden inaktiv ist.

Der Polymerisationsgrad der so erhaltenen zumindest teilweise polymerisierten Voremulsion beträgt typischerweise mehr als 80 %, insbesondere mehr als 95 %, besonders bevorzugt mehr als 99 %. Zu der zumindest teilweise polymerisierten Voremulsion gibt man dann die Restmenge der Monomerzusammensetzung, vorzugsweise in Form einer Monomeremulsion, vollständig zu und initiiert eine radikalische Polymerisation.

Die Polymerisation kann auf verschiedene Weise initiiert werden. So kann man einen thermisch aktivierbaren radikalischen Initiator verwenden, wie die oben genannten. Nicht verbrauchter thermisch aktivierbarer Initiator aus dem Schritt (i) kann ausreichend sein oder zur Initiierung beitragen. Bei Einsatz eines thermisch aktivierbaren radikalischen Initiators erfolgt die Initiierung gegebenenfalls durch Erhitzen auf Polymerisationstemperatur.

Erfindungsgemäβ bedient man sich eines Redoxinitiatorsystems zur Initiierung der Polymerisation im Schritt (ii).

Ein Redoxinitiatorsystem umfasst wenigstens eine Oxidationsmittelkomponente und wenigstens eine Reduktionsmittelkomponente. Geeignete Oxidationsmittelkomponenten sind beispielsweise Peroxide und/oder Hydroperoxide wie Wasserstoffperoxid, tert.-Butylperoxid, Cumolhydroperoxid, Pinanhydroperoxid, Diisopropylphenylhydroperoxid, Dibenzoylperoxid, Dilauroylperoxid und Diacetylperoxid. Wasserstoffperoxid ist bevorzugt.

Geeignete Reduktionsmittelkomponenten sind beispielsweise Cer-, Mangan- oder Eisen(II)-salze, Alkalimetallsulfite, Ascorbinsäure, Acetonbisulfit-Addukt und/oder ein Alkalimetallsalz der Hydroxymethansulfinsäure. Eine Kombination von Eisen(II)-salzen und Ascorbinsäure ist bevorzugt.

In einer geeigneten Ausführungsform initiiert man im Schritt (ii) die radikalische Polymerisation durch Zugabe einer Reduktionsmittelkomponente eines Redoxinitiatorsystems. In diesem Fall hat man zuvor eine Oxidationsmittelkomponente eines Redoxinitiatorsystems zugegeben. Alternativ kann auch überschüssiger Initiator aus dem Schritt (i) als eine Oxidationsmittelkomponente eines Redoxinitiatorsystems fungieren. Die Zugabe der Reduktionsmittelkomponente kann auf verschiedene Weise erfolgen. Man kann die Reduktionsmittelkomponente z. B. auf einmal oder als kontinuierlichen Zulauf über eine Zeitspanne verteilt zugeben.

In einer anderen geeigneten Ausführungsform initiiert man im Schritt (ii) die radikalische Polymerisation durch im Wesentlichen gleichzeitige Zugabe einer Oxidationsmittelkomponente eines Redoxinitiators und einer Reduktionsmittelkomponente eines Redoxinitiators. Der Initiator bzw. die Komponenten des Redoxinititorsystems werden in ausreichender Menge verwendet, um die Polymerisationsreaktion zu initiieren.

Es kann zweckmäßig sein, im Schritt (ii) die zumindest teilweise polymerisierten Voremulsion in Gegenwart der Restmenge der Monomerzusammensetzung wenigstens eine Stunde, z. B. 2 bis 24 Stunden, quellen zu lassen, bevor man die radikalische Polymerisation initiiert.

In den meisten Fällen wird man im Schritt (ii) die Restmenge der Monomerzusammensetzung innerhalb einer Zeitspanne von weniger als einer Stunde zu der zumindest teilweise polymerisierten Voremulsion zugeben und nach vollständiger Zugabe die radikalische Polymerisation unverzüglich initiieren.

Die Monomerzusammensetzung enthält einen ausreichenden Anteil an ethylenisch ungesättigten Carbonsäuren, dass das erhaltene Copolymer alkalilöslich ist. Andererseits enthält die Monomerzusammensetzung einen ausreichenden Anteil an ethylenisch ungesättigten hydrophoben Monomeren, dass das erhaltene Copolymer im sauren und neutralen pH-Bereich im Wesentlichen wasserunlöslich ist und eine vergleichsweise niedrigviskose Dispersion bildet. Das im alkalischen Bereich gelöste Copolymer bildet vorzugsweise eine hochtransparente Lösung, insbesondere in Gegenwart von Tensiden, zu denen die hydrophoben Bestandteile des gelösten polymeren Verdickers eine hohe Verträglichkeit vermitteln. Vorzugsweise ist die Zusammensetzung des Monomerengemisches im Schritt (i) und im Schritt (ii) identisch. Die Zusammensetzungen können aber auch voneinander abweichen, indem die Konzentrationen einzelner oder mehrerer Monomere in den Schritten (i) und (ii) relativ zueinander verändert werden oder bestimmte Monomere nur in einem der Schritte (i) oder (ii) eingesetzt werden. In jedem Fall ist die Monomerzusammensetzung im Schritt (i) und im Schritt (ii) jeweils so beschaffen, dass sowohl das Voremulsionspolymer als auch das fertige Copolymer alkalilöslich sind, im sauren und neutralen pH-Bereich aber unlöslich sind.

Das ethylenisch ungesättigte hydrophobe Monomer ist vorzugsweise ausgewählt unter C₁-C₉-Alkyl(meth)acrylaten, Dienen, Vinylaromaten, (Meth)acrylnitril und Gemischen davon.

Der Alkylrest in den C₁-C₉-Alkyl(meth)acrylaten kann linear oder verzweigt sein. Geeignete C₁-C₉-Alkyl(meth)acrylate sind beispielsweise Methyl(meth)acrylat, Ethyl-(meth)acylat, n-Propyl(meth)acrylat, n-Butyl(meth)acrylat, Ethylhexl(meth)acrylat, Nonyl(meth)crylat, Isonony(meth)acrylat.

Geeignete Diene sind vorzugsweise konjugierte C₄-C₈-Diene wie insbesondere Butadien und Isopren.

Geeignete Vinylaromaten sind Styrol und Methylstyrole.

In bevorzugten Ausführungsformen umfasst das ethylenisch ungesättigte hydrophobe Monomer:
b1) wenigstens ein C₁-C₂-Alkylmethacrylat, und
b2) wenigstens ein C₂-C₉-Alkylacrylat, insbesondere C₂-C₄-Alkylacrylat, wobei die über die Zahl der Alkylgruppen des Alkylacrylats gemittelte Alkylkettenlänge wenigstens 2,1 beträgt.

Das Gewichtsverhältnis b1) : b2) beträgt vorzugsweise 2:1 bis 1:15.

Geeignete C₁-C₂-Alkylmethacrylate sind Methylmethacrylat und Ethylmethacylat, wovon Methylmethacrylat besonders bevorzugt ist.

Geeignete C₂-C₉-Alkylacrylate sind Ethylacrylat, n-Propylacrylat, n-Butylacrylat, Ethylhexylacrylat, Nonylacrylat und Isononylacrylat. Die Art und Menge der C₂-C₄-Alkylacrylate werden so gewählt, dass sich eine bestimmte über die Zahl der Alkylgruppen der C₂-C₉-Alkylacrylateinheiten mittlere Alkylkettenlänge einstellt. Vorzugsweise beträgt die gemittelte Alkylkettenlänge wenigstens 2,1, insbesondere 2,1 bis 4,0. Die mittlere Alkylkettenlänge wird berechnet, indem man die Kohlenstoffzahl der längsten Alkylkette des Alkylrests (d. h. beispielsweise 2 für Ethyl und 4 für n-Butyl) mit dem molaren Anteil des Alkylacrylats an der Gesamtmenge der C₂-C₉-Alkylacrylate multipliziert und die einzelnen Beiträge addiert.

Vorzugsweise umfasst das C₂-C₉-Alkylacrylat zumindest n-Butylacrylat, insbesondere ein Gemisch von n-Butylacrylat mit Ethylacrylat. Vorzugsweise enthält das Copolymer 5 bis 85 Gew.-%, bezogen auf das Gesamtgewicht des Copolymers, einpolymerisierte Einheiten von n-Butylacrylat, wobei ein Bereich von über 10 Gew.-% bis 60 Gew.-% bevorzugt und ein Bereich von 15 Gew.-% bis 45 Gew.-% besonders bevorzugt ist.

In bevorzugten Ausführungsformen umfasst die Monomerzusammensetzung außerdem wenigstens ein ethylenisch ungesättigtes Assoziativmonomer. Assoziativmonomer weisen hydrophobe oder tensidartige Seitengruppen auf, welche in der Lage sind, in einem hydrophilen Medium sowohl mit sich selbst als auch mit anderen hydrophoben Stoffen, wie Tensidmicellen, Pigmenten, Bindemittelteilchen oder Füllstoffen, in Wechselwirkung zu treten und nichtkovalente Netzwerke zu bilden.

Das ethylenisch ungesättigte Assoziativmonomer ist beispielsweise ausgewählt unter C₁₀-C₃₀-Alkyl(meth)acrylaten und ethylenisch ungesättigten Tensidmonomeren.

Nichtionische ethylenisch ungesättigte Tensidmonomere, die sich als Assoziativmonomer eignen, sind an sich bekannt. Es handelt sich z. B. um
(a) Urethangruppen-haltige Umsetzungsprodukte eines monoethylenisch ungesättigten Isocyanats und nichtionischer Tenside
(b) Ester ethylenisch ungesättigter Carbonsäuren und nichtionischer Tenside,
(c) Allylether nichtionischer Tenside.

Als nichtionische Tenside eignen sich vorzugsweise alkoxylierte C₆-C₃₀-Alkohole wie Fettalkoholalkoxylate oder Oxoalkoholalkoxylate. Pro Mol Alkohol verwendet man wenigstens 2, z. B. 2 bis 100, vorzugsweise 3 bis 20 Mol mindestens eines C₂-C₄-Alkylenoxids. Unterschiedliche Alkylenoxideinheiten können blockweise angeordnet oder statistisch verteilt vorliegen. Vorzugsweise setzt man als Alkylenoxid Ethylenoxid und/oder Propylenoxid ein.

Eine weitere Klasse geeigneter nichtionischer Tenside sind Alkylphenolethoxylate mit C₆-C₁₄-Alkylketten und 5 bis 30 Mol Ethylenoxideinheiten.

In bevorzugten Ausführungsformen weist das nichtionische ethylenisch ungesättigte Tensidmonomer die allgemeine Formel auf

R-O-(CH₂CHR'-O)ₙ-CO-CR"=CH₂

worin R für C₆-C₃₀-Alkyl, vorzugsweise C₈-C₂₂-Alkyl, steht,
R' für Wasserstoff oder Methyl steht, vorzugsweise für Wasserstoff,
R" für Wasserstoff oder Methyl steht, vorzugsweise für Methyl, und
n für eine ganze Zahl von 2 bis 100, vorzugsweise 3 bis 50, steht.

Die Wiederholungseinheiten in der Klammer leiten sich von Ethylenoxid oder Propylenoxid ab. Die Bedeutung von R' ist in jeder Wiederholungseinheit unabhängig von anderen Wiederholungseinheiten. Unterschiedliche Alkylenoxideinheiten können blockweise angeordnet oder statistisch verteilt vorliegen.

Die Monomerzusammensetzung kann außerdem ein mehrfach ethylenisch ungesättigtes Monomer umfassen. Verwendbare mehrfach ethylenisch ungesättigte Monomere sind z. B. Ethylenglycoldi(meth)acrylat, 1,6-Hexandioldi(meth)acrylat, Divinylbenzol und dergleichen.

Bei der ethylenisch ungesättigten Carbonsäure handelt es sich im Allgemeinen um eine monoethylenisch ungsättigte Mono- oder Dicarbonsäure mit 3 bis 8 Kohlenstoffatomen. Geeignete ethylenisch ungesättigte Carbonsäuren sind z. B. ausgewählt unter Acrylsäure, Methacrylsäure, Itaconsäure und Maleinsäure. Davon ist Methacrylsäure besonders bevorzugt.

Typischerweise umfasst die Monomerzusammensetzung:
a) 10 bis 75 Gew.-%, vorzugsweise 20 bis 50 Gew.-%, ethylenisch ungesättigte Carbonsäure,
b) 5 bis 90 Gew.-%, vorzugsweise 15 bis 80 Gew.-%, ethylenisch ungesättigtes hydrophobes Monomer,
c) 0 bis 50 Gew.-%, vorzugsweise 1 bis 10 Gew.-%, ethylenisch ungesättigtes Assoziativmonomer, und
d) 0 bis 9 Gew.% mehrfach ethylenisch ungesättigtes Monomer.

Die Herstellung der zumindest teilweise polymerisierten Voremulsion und/oder die Polymerisation der Restmenge der Monomerzusammensetzung erfolgt in der Regel in Gegenwart eines anionischen und/oder nichtionischen Emulgators.

Typische Emulgatoren sind anionische Emulgatoren wie z. B. Natriumlaurylsulfat, Natriumtridecylethersulfate, Dioctylsulfosuccinat Natriumsalz und Natriumsalze von Alkylarylpolyethersulfonaten; und nichtionische Emulgatoren wie z. B. Alkylarylpolyetheralkohole und Ethylenoxid-Propylenoxid-Copolymere.

Bevorzugte Emulgatoren weisen die allgemeine Formel auf

R-O-(CH₂-CHR'-O)ₙ-X

worin R für C₆-C₃₀-Alkyl steht,
R' für Wasserstoff oder Methyl steht,
X für Wasserstoff oder SO₃M steht,
M für Wasserstoff oder ein Alkalimetall steht, und
n für eine Zahl von 2 bis 100 steht.

Die Copolymerdispersion kann einer chemischen Desodorierung unterzogen werden. Bei der chemischen Desodorierung wird nach Ende der eigentlichen Emulsionspolymerisation weiterer Initiator, z.B. ein Redoxinitiator zugesetzt. Zur chemischen Desodorierung geeignete Redoxinitiatoren umfassen als oxidierende Komponente beispeilsweise wenigstens ein organisches Peroxid und/oder Hydroperoxid wie Wasserstoffperoxid, tert.-Butylperoxid, Cumolhydroperoxid, Pinanhydroperoxid, Diisopropylphenylhydroperoxid, Dibenzoylperoxid, Dilauroylperoxid und Diacetylperoxid und als reduzierend wirkende Komponente beispielsweise Alkalimetallsulfite, Ascorbinsäure, Acetonbisulfit-Addukt und/oder ein Alkalimetallsalz der Hydroxymethansulfinsäure, wobei Eisen(II)-salze, vorzugsweise in komplexierter Form, als Katalysator zugesetzt werden können.

Die Copolymerdispersion weist in der Regel einen Feststoffgehalt von 25 bis 70 Gew.-%, insbesondere etwa 30 bis 50 Gew.-%, auf.

In unneutralisierter Form weist die Copolymerdispersion eine relativ niedrige Viskosität auf. Sie ist daher leicht handhabbar und kann problemlos dosiert oder umgepumpt werden. Durch Neutralisation, z. B. auf einen pH von mehr als 5, vorzugsweise mehr als 6, insbesondere 8 bis 10, wird das Copolymer löslich und die Viskosität des wässrigen Mediums steigt stark an. Dabei werden vorzugsweise mehr als 50 Mol-% der Säuregruppen im Verdicker neutralisiert. Geeignete Neutralisierungsmittel sind z. B. Natriumhydroxid, Kaliumhydroxid, Ammoniumhydroxid, Amine, wie Triethylamin, Triethanolamin, Monoethanolamin, und andere alkalische Materialien.

Die erfindungsgemäßen Verdicker eignen sich zur Rheologiemodifizierung von Papier-Streichmassen, Textildruckpasten, Arzneimitteln, kosmetischen Mitteln, Waschmitteln, Reinigungsmitteln oder Nahrungsmitteln. Besonders bevorzugt ist die Verwendung in flüssigen Wasch- und Reinigungsmitteln, insbesondere transparenten flüssigen Wasch- oder Reinigungsmittelzusammensetzungen.

Neben dem Verdicker enthalten die flüssigen Wasch- oder Reinigungsmittel Tensid(e), wobei anionische, nichtionische, kationische und/oder amphotere Tenside eingesetzt werden können. Bevorzugt sind aus anwendungstechnischer Sicht Mischungen aus anionischen und nichtionischen Tensiden. Der Gesamttensidgehalt des flüssigen Wasch- oder Reinigungsmittel beträgt vorzugsweise 5 bis 60 Gew.-% und besonders bevorzugt 15 bis 40 Gew.-%, bezogen auf das gesamte flüssige Wasch- oder Reinigungsmittel.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂-C₁₄-Alkohole mit 3 EO, 4 EO oder 7 EO, C₉-C₁₁-Alkohol mit 7 EO, C₁₃-C₁₅-Alkohole mit 3 EO, 5 EO, 7 EO oder 8 EO, C₁₂-C₁₈-Alkohole mit 3 EO, 5 EO oder 7 EO und Mischungen aus diesen, wie Mischungen aus C₁₂-C₁₄-Alkohol mit 3 EO und C₁₂-C₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside der allgemeinen Formel (1)

RO(G)ₓ (1)

eingesetzt werden, worin R für einen primären geradkettigen oder methylverzweigten, insbesondere in 2-Stellung methylverzweigten aliphatischen Rest mit 8 bis 22, vorzugsweise 12 bis 18 C-Atomen steht und G für eine Glykosideinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (2), 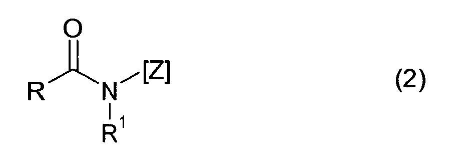 worin RC(=O) für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (3) 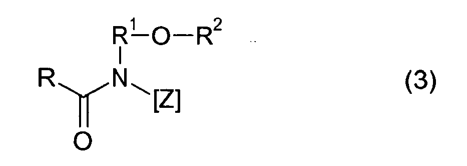 worin R für einen linearen oder verzweigten Alkyl- oder Alkenylrest mit 7 bis 12 Kohlenstoffatomen, R¹ für einen linearen, verzweigten oder cyclischen Alkylenrest mit 2 bis 8 Kohlenstoffatomen oder einen Arylenrest mit 6 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁-C₄-Alkyl- oder Phenylreste bevorzugt sind, und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines Zuckers erhalten, beispielsweise Glucose, Fructose, Maltose, Lactose, Galactose, Mannose oder Xylose. Die N-Alkoxyoder N-Aryloxy-substituierten Verbindungen können dann beispielweise gemäß WO-A-95/07331 durch Umsetzung mit Fettsäuremethylestern in Gegenwart eines Alkoxids als Katalysator in die gewünschten Polyhydroxyfettsäureamide überführt werden.

Der Gehalt an nichtionischen Tensiden beträgt in den flüssigen Wasch- oder Reinigungsmitteln bevorzugt 5 bis 30 Gew.-%, vorzugsweise 7 bis 20 Gew.-% und insbesondere 9 bis 15 Gew.- %, jeweils bezogen auf das gesamte Mittel.

Als anionische Tenside werden beispielsweise solche vom Typ der Sulfonate und Sulfate eingesetzt. Als Tenside vom Sulfonat-Typ kommen dabei vorzugsweise C₉-C₁₃-Alkylbenzolsulfonate, Olefinsulfonate, d.h. Gemische aus Alken- und Hydroxyalkansulfonaten sowie Disulfonaten, wie man sie beispielsweise aus C₁₂-C₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂-C₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Taigfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten be-sitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN® erhalten werden können, sind geeignete Aniontenside.

Auch die Schwefelsäuremonoester der mit 1 bis 6 Mol Ethylenoxid ethoxylierten geradkettigen oder verzweigten C₇-C₂₁-Alkohole, wie 2-Methyl-verzweigte C₉-C₁₁-Alkohole mit im Durchschnitt 3,5 Mol Ethylenoxid (EO) oder C₁₂-C₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈-C₁₈- Fettalkoholreste oder Mischungen aus diesen. Insbesondere bevorzugte Sulfosuccinate enthalten einen Fettalkoholrest, der sich von ethoxylierten Fettalkoholen ableitet. Dabei sind wiederum Sulfosuccinate, deren Fettalkohol-Reste sich von ethoxylierten Fettalkoholen mit engerr Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Taigfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Der Gehalt bevorzugter flüssiger Wasch- oder Reinigungsmittel an anionischen Tensiden beträgt 2 bis 30 Gew.-%, vorzugsweise 4 bis 25 Gew.-% und insbesondere 5 bis 22 Gew.-%, jeweils bezogen auf das gesamte Mittel. Es ist besonders bevorzugt, dass die Menge an Fettsäureseife mindestens 2 Gew.-% und besonders bevorzugt mindestens 4 Gew.-% und insbesondere bevorzugt mindestens 6 Gew.-% beträgt.

Die Viskosität der flüssigen Wasch- oder Reinigungsmittel kann mit üblichen Standardmethoden (beispielsweise Brookfield-Viskosimeter LVT-II bei 20 U/min und 20 °C, Spindel 3) gemessen werden und liegt vorzugsweise im Bereich von 100 bis 5000 mPas. Bevorzugte Mittel haben Viskositäten von 300 bis 4000 mPas, wobei Werte zwischen 1000 und 3000 mPas besonders bevorzugt sind.

Zusätzlich zu dem Verdicker und zu dem/den Tensid(en) können die flüssigen Wasch- oder Reinigungsmittel weitere Inhaltsstoffe enthalten, die die anwendungstechnischen und/oder ästhetischen Eigenschaften des flüssigen Wasch- oder Reinigungsmittels weiter verbessern. In der Regel enthalten bevorzugte Mittel zusätzlich zu dem Verdicker und zu Tensid(en) einen oder mehrere Stoffe aus der Gruppe der Gerüststoffe, Bleichmittel, Bleichaktivatoren, Enzyme, Elektrolyte, nichtwässrigen Lösungsmittel, pH-Stellmittel, Duftstoffe, Parfümträger, Fluoreszenzmittel, Farbstoffe, Schauminhibitoren, Hydrotope, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Korrosionsinhibitoren, Antistatika, Bügelhilfsmittel, Phobier- und Imprägniermittel, Quell- und Schiebefestmittel sowie UV-Absorber.

Als Gerüststoffe bzw. Builder, die in den flüssigen Wasch- oder Reinigungsmitteln enthalten sein können, sind insbesondere Silikate, Aluminiumsilikate (insbesondere Zeolithe), sowie besonders bevorzugt bei transparenten Flüssigwaschmitteln Carbonate, Salze organischer Di- und Polycarbonsäuren sowie Mischungen dieser Stoffe zu nennen.

Geeignete niedermolekulare Polycarboxylate als organische Builder sind beispielsweise:
C₄-C₂₀-Di-, -Tri- und -Tetracarbonsäuren wie z.B. Bernsteinsäure, Propantricarbonsäure, Butantetracarbonsäure, Cyclopentantetracarbonsäure und Alkyl- und Alkylenbernsteinsäuren mit C₂-C₁₆-Alkyl- bzw. -Alkylen-Resten;
C₄-C₂₀-Hydroxycarbonsäuren wie z.B. Äpfelsäure, Weinsäure, Gluconsäure, Glutarsäure, Citronensäure, Lactobionsäure und Saccharosemono-, -di- und -tricarbonsäure;
Aminopolycarboxylate wie z.B. Nitrilotriessigsäure, Methylglycindiessigsäure, Alanindiessigsäure, Ethylendiamintetraessigsäure und Serindiessigsäure;
Salze von Phosphonsäuren wie z.B. Hydroxyethandiphosphonsäure, Ethylendiamintetra(methylenphoshponat) und Diethylentriaminpenta(methylenphosphat).

Geeignete oligomere oder polymere Polycarboxylate als organische Builder sind beispielsweise:
Oligomaleinsäuren, wie sie beispielsweise in EP-A 0 451 508 und EP-A 0 396 303 beschrieben sind;
Co- und Terpolymere ungesättigter C₄-C₈-Dicarbonsäuren, wobei als Comonomere monoethylenisch ungesättigte Monomere
aus der Gruppe (i) in Mengen von bis zu 95 Gew.-%
aus der Gruppe (ii) in Mengen von bis 60 Gew.-%
aus der Gruppe (iii) in Mengen von bis zu 20 Gew.-%
einpolymerisiert enthalten sein können.

Als ungesättigte C₄-C₈-Dicarbonsäuren sind hierbei beispielsweise Maleinsäure, Fumarsäure, Itaconsäure und Citraconsäure geeignet. Bevorzugt ist Maleinsäure.

Die Gruppe (i) umfasst monoethylenisch ungesättigte C₃-C₈-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfasst monoethylenisch ungesättigte C₂-C₂₂-Olefine, Vinylalkylether mit C₁-C₈-Alkylgruppen, Styrol, Vinylester von C₁-C₈-Carbonsäure, (Meth)acrylamid und Vinylpyrrolidon. Bevorzugt werden aus der Gruppe (ii) C₂-C₆-Olefine, Vinylalkylether mit C₁-C₄-Alkylgruppen, Vinylacetat und Vinylpropionat eingesetzt.

Die Gruppe (iii) umfasst (Meth)acrylester von C₁-C₈-Alkoholen, (Meth)acrylnitril, (Meth)acrylamide, (Meth)acrylamide von C₁-C₈-Aminen, N-Vinylformamid und Vinylimidazol.

Falls die Polymeren der Gruppe (ii) Vinylester einpolymerisiert enthalten, können dieses auch teilweise oder vollständig zu Vinylalkohol-Struktureinheiten hydrolysiert vorliegen. Geeignete Co- und Terpolymere sind beispielsweise aus US 3,887,806 sowie SE-A 43 13 909 bekannt.

Als Copolymere von Dicarbonsäuren eignen sich als organische Builder vorzugsweise:

Copolymere von Maleinsäure und Acrylsäure im Gewichtsverhältnis 10:90 bis 95:5, insbesondere bevorzugt solche im Gewichtsverhältnis 30:70 bis 90:10 mit Molmassen von 10000 bis 150000;

Terpolymere aus Maleinsäure, Acrylsäure und einem Vinylester einer C₁-C₃-Carbonsäure im Gewichtsverhältnis 10 (Maleinsäure) : 90 (Acrylsäure + Vinylester) bis 95 (Maleinsäure) : 10 (Acrylsäure + Vinylester), wobei das Gew.-Verhältnis von Acrylsäure zu Vinylester im Bereich von 20:80 bis 80:20 variieren kann, und besonders bevorzugt

Terpolymere aus Maleinsäure, Acrylsäure und Vinylacetat oder Vinylpropionat im Gewichtsverhältnis 20 (Maleinsäure) : 80 (Acrylsäure + Vinylester) bis 90 (Maleinsäure) : 10 (Acrylsäure + Vinylester), wobei das Gew.-Verhältnis von Acrylsäure zum Vinylester im Bereich von 30:70 bis 70:30 variieren kann;

Copolymere von Maleinsäure mit C₂-C₈-Olefinen im Molverhältnis 40:60 bis 80:20, wobei Copolymere von Maleinsäure mit Ethylen, Propylen oder Isobutan im Molverhältnis 50:50 besonders bevorzugt sind.

Pfropfpolymere ungesättigter Carbonsäuren auf niedermolekulare Kohlenhydrate oder hydrierte Kohlenhydrate, vgl. US 5,227,446, DE-A 44 15 623, DE-A 43 13 909, sind ebenfalls als organische Builder geeignet.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z.B. saure oder enzymatisch abgebaute Stärken, Inuline oder Zellulose, reduzierte (hydrierte oder hydrierend aminierte) abgebaute Polysaccharide wie z.B. Mannit, Sorbit, Aminosorbit und Glucamin geeignet sowie Polyalkylenglycole mit Molmassen bis zu Mw = 5000 wie z.B. Polyethylenglycole, Ethylenoxid/Propylenoxid- bzw. Ethylenoxid/Butylenoxid-Blockcopolymere, statistische Ethylenoxid/Popylenoxid- beziehungsweise Ethylenoxid/Butylenoxid-Copolymere, alkoxylierte ein- oder mehrbasische C₁-C₂₂-Alkohole, vgl. US 4,746,456.

Bevorzugt werden aus dieser Gruppe gepfropfte abgebaute beziehungsweise abgebaute reduzierte Stärken und gepfropfte Polyethylenoxide eingesetzt, wobei 20 bis 80 Gew.-% Monomere bezogen auf die Pfropfkomponente bei der Pfropfpolymerisation eingesetzt werden. Zur Pfropfung wird vorzugsweise eine Mischung von Maleinsäure und Acrylsäure im Gew.-Verhältnis von 90:10 bis 10:90 eingesetzt.

Polyglyoxylsäuren als organische Builder sind beispielsweise beschrieben in EP-B 0 001 004, US 5,399,286, DE-A 41 06 355 und EP-A 0 656 914. Die Endgruppen der Polyglyoxylsäuren können unterschiedliche Strukturen aufweisen.

Polyamidocarbonsäuren und modifizierte Polyamidocarbonsäuren als organische Builder sind beispielsweise bekannt aus EP-A 0 454 126, EP-B 0 511 037, WO-A 94/01486 und EP-A 0 581 452.

Vorzugsweise verwendet man als organische Builder auch Polyasparaginsäure oder Cokondensate der Asparaginsäure mit weiteren Aminosäuren, C₄-C₂₅-Mono- oder - Dicarbonsäuren und/oder C₄-C₂₅-Mono- oder -Diaminen. Besonders bevorzugt werden in phosphorhaltigen Säuren hergestellte, mit C₆-C₂₂-Mono- oder -Dicarbonsäuren beziehungsweise mit C₆-C₂₂-Mono- oder -Diaminen modifizierte Polyasparaginsäuren eingesetzt.

Kondensationsprodukte der Citronensäure mit Hydroxycarbonsäuren oder Polyhydroxyverbindungen als organische Builder sind z.B. bekannt aus WO-A 93/22362 und WO-A 92/16493. Solche Carboxylgruppen enthaltende Kondensate haben üblicherweise Molmassen bei zu 10000, vorzugsweise bis zu 5000.

Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure.

Um beim Waschen bei Temperaturen von 60 °C und darunter eine verbesserte Bleichwirkung zu erreichen, können Bleichaktivatoren in die Wasch- oder Reinigungsmittel eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1 ,3,5-triazin (DADHT), acylierte Glykolurile, insbesondere Tetraacetylglykoluril (TAGU)1 N-Acylimide, insbesondere N-Nonanoylsuccinimid (NOSI), acylierte Phenolsulfonate, insbesondere n-Nonanoyl- oder Isononanoyloxybenzolsulfonat (nbzw. iso-NOBS), Carbonsäureanhydride, insbsondere Phthalsäureanhydrid, acylierte mehrwertige Alkohole, insbesondere Triacetin, Ethylenglykoldiacetat und 2,5-Diacetoxy-2,5- dihydrofuran.

Zusätzlich zu den konventionellen Bleichaktivatoren oder an deren Stelle können auch sogenannte Bleichkatalysatoren in die flüssigen Wasch- oder Reinigungsmittel eingearbeitet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit stickstoffhaltigen Tripod-Liganden sowie Co-, Fe-, Cu- und Ru-Aminkomplexe sind als Bleichkatalysatoren verwendbar.

Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen wie protein-, fett- oder stärkehaltigen Verfleckungen und Vergrauungen bei. Cellulasen und andere Glykosylhydrolasen können darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich verschiedene Cellulase-Typen durch ihre CMCaseund Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,12 bis etwa 2,5 Gew.-% betragen.

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Mitteln bevorzugt. Der Anteil an Elektrolyten in den Mitteln beträgt üblicherweise 0,5 bis 5 Gew.-%.

Nichtwässrige Lösungsmittel, die in den flüssigen Wasch- oder Reinigungsmitteln eingesetzt werden können, stammen beispielsweise aus der Gruppe ein- oder mehrwertigen Alkohole, Alkanolamine oder Glykolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propylether, Dipropylenglykolmonomethyl- oder - ethylether, Di-isopropylenglykolmonomethyloder -ethylether, Methoxy-, Ethoxyoder Butoxytriglykol, i-Butoxy-ethoxy-2-propanol, 3-Methyl-3-methoxybutanol, Propylen-glykol-t-butylether sowie Mischungen dieser Lösungsmittel. Nichtwässrige Lösungsmittel können in den flüssigen Wasch- oder Reinigungsmitteln in Mengen zwischen 0,5 und 15 Gew.-%, bevorzugt aber unter 12 Gew.-% und insbesondere unterhalb von 9 Gew.-% eingesetzt werden.

Um den pH-Wert der flüssigen Wasch- oder Reinigungsmittel in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 7 Gew.-% der Gesamtformulierung nicht.

Um den ästhetischen Eindruck der flüssigen Wasch- oder Reinigungsmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Als Schauminhibitoren, die in den flüssigen Wasch- oder Reinigungsmitteln eingesetzt werden können, kommen beispielsweise Seifen, Paraffine oder Silikonöle in Betracht, die gegebenenfalls auf Trägermaterialien aufgebracht sein können.

Geeignete Antiredepositionsmittel, die auch als "soil repellents" bezeichnet werden, sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether. Geeignete Soil-Release-Polymere sind beispielsweise Polyester aus Polyethylenoxiden mit Ethylenglycol und/oder Propylenglycol und aromatischen Dicarbonsäuren oder aromatischen und aliphatischen Dicarbonsäuren; Polyester aus einseitig endgruppenverschlossenen Polyethylenoxiden mit zwei- und/oder mehrwertigen Alkoholen und Dicarbonsäure, insbesondere Polymere aus Ethylenterephthalaten und/oder Polyethylenglycolterephthalaten oder anionisch und/oder nichtionisch modifizierten Derivaten von diesen. Insbesondere bevorzugt von diesen sind die sulfonierten Derivate der Phthalsäure- und Terephthalsäure-Polymere. Derartige Polyester sind bekannt, beispielsweise aus US 3,557,039, GB-A 11 54 730, EP-A 0 185 427, EP-A 0 241 984, EP-A 0 241 985, EP-A 0 272 033 und US-A 5,142,020. Weitere geeignete Soil-Release-Polymere sind amphiphile Pfopf- oder Copolymere von Vinyl- und/oder Acrylestern auf Polyalkylenoxide (vgl. US 4,746,456, US 4,846,995, DE-A 37 11 299, US 4,904,408, US 4,846,994 und US 4,849,126) oder modifizierte Cellulosen wie z.B. Methylcellulose, Hydroxypropylcellulose oder Carboxymethylcellulose.

Optische Aufheller (sogenannte "Weißtöner") können den flüssigen Wasch- oder Reinigungsmitteln zugesetzt werden, um Vergrauungen und Vergilbungen der behandelten Textilen Flächengebilden zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3- Diarylpyrazoline, Naphthalsäureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0,03 und 0,3 Gew.-%, bezogen auf das fertige Mittel, eingesetzt.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, zum Beispiel abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxymethylcellulose und deren Gemische in Mengen von 0,1 bis 5 Gew.-%, bezogen auf die Mittel, eingesetzt.

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Mittel synthetische Knitterschutzmittel enthalten. Hierzu zählen beispielsweise synthetische Produkte auf der Basis von Fettsäuren, Fettsäureestern, Fettsäureamiden, Fettalkylolestern, Fettalkylolamiden oder Fettalkoholen, die meist mit Ethylenoxid umgesetzt sind, oder Produkte auf der Basis von Lecithin oder modifizierter Phosphorsäureester.

Zur Bekämpfung von Mikroorganismen können die flüssigen Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat.

Um unerwünschte, durch Sauerstoffeinwirkung und andere oxidative Prozesse verursachte Veränderungen an den flüssigen Wasch- oder Reinigungsmitteln und/oder den behandelten textilen Flächengebilden zu verhindern, können die Mittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite und Phosphonate.

Ein erhöhter Tragekomfort kann aus der zusätzlichen Verwendung von Antistatika resultieren, die den Mitteln zusätzlich beigefügt werden. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Externe Antistatika sind beispielsweise in den Patentanmeldungen FR 1,156,513, GB 873 214 und GB 839 407 beschrieben. Die hier offenbarten Lauryl-(bzw. Stearyl-) dimethylbenzylammoniumchloride eignen sich als Antistatika für textile Flächengebilde bzw. als Zusatz zu Waschmitteln, wobei zusätzlich ein Avivageeffekt erzielt wird.

Zur Verbesserung des Wasserabsorptionsvermögens, der Wiederbenetzbarkeit der behandelten textilen Flächengebilde und zur Erleichterung des Bügelns der behandelten textilen Flächengebilde können in den flüssigen Wasch- oder Reinigungsmitteln beispielsweise Silikonderivate eingesetzt werden. Diese verbessern zusätzlich das Ausspülverhalten der Mittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H und/oder Si-CI-Bindungen aufweisen. Die Viskositäten der bevorzugten Silikone liegen bei 25 °C im Bereich zwischen 100 und 100.000 mPas, wobei die Silikone in Mengen zwischen 0,2 und 5 Gew.-%, bezogen auf das gesamte Mittel eingesetzt werden können.

Schließlich können die flüssigen Wasch- oder Reinigungsmittel auch UV-Absorber enthalten, die auf die behandelten textilen Flächengebilde aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner sind beispielsweise die Alkalisalze der Ethylendiamintetraessigsäure (EDTA), der Nitrilotriessigsäure (NTA) oder Methylglycindiessigsäure (MGDA) sowie Alkalimetallsalze von anionischen Polyelektrolyten wie Polymaleaten und Polysulfonaten.

Eine bevorzugte Klasse von Komplexbildnern sind die Phosphonate, die in bevorzugten flüssigen Wasch- oder Reinigungsmitteln in Mengen von 0,01 bis 2,5 Gew.-%, vorzugsweise 0,02 bis 2 Gew.-% und insbesondere von 0,03 bis 1 ,5 Gew.-% enthalten sind. Zu diesen bevorzugten Verbindungen zählen insbesondere Organophosphonate wie beispielsweise 1-Hydroxyethan-1 ,1-diphosphonsäure (HEDP), Amnotri(methylen-phosphonsäure) (ATMP), Diethylentriamin-penta(methylenphosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

Die erhaltenen wässrigen flüssigen Wasch- oder Reinigungsmittel weisen keinen Bodensatz auf; in einer bevorzugten Ausführungsform sind sie transparent oder zumindest transluzent. Vorzugsweise weisen die wässrigen flüssigen Wasch- oder Reinigungsmittel eine Transmission des sichtbaren Lichtes von mindestens 30%, vorzugsweise 50%, insbesondere bevorzugt 75%, am meisten bevorzugt 90% auf. Alternativ können die erfindungsgemäßen Verdicker in opake Wasch- oder Reinigungsmittel eingearbeitet werden.

Neben diesen Bestandteilen kann ein wässriges Wasch- oder Reinigungsmittel dispergierte Partikel, deren Durchmesser entlang ihrer größten räumlichen Ausdehnung 0,01 bis 10.000 µm beträgt, enthalten.

Partikel können Mikrokapseln als auch Granulate, Compounds und Duftperlen sein, wobei Mikrokapseln bevorzugt sind.

Unter dem Begriff "Mikrokapsel" werden Aggregate verstanden, die mindestens einen festen oder flüssigen Kern enthalten, der von mindestens einer kontinuierlichen Hülle, insbesondere einer Hülle aus Polymer(en), umschlossen ist. Üblicherweise handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Bevorzugt sind einkernige Mikrokapseln mit einer kontinuierlichen Hülle. Die Hülle kann aus natürlichen, halbsynthetischen oder synthetischen Materialien bestehen. Natürlich Hüllmaterialien sind beispielsweise Gummi arabicum, Agar Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Im Inneren der Mikrokapseln können empfindliche, chemisch oder physikalisch inkompatible sowie flüchtige Komponenten (= Wirkstoffe) des wässrigen flüssigen Wasch- oder Reinigungsmittels lager- und transportstabil eingeschlossen werden. In den Mikrokapseln können sich beispielsweise optische Aufheller, Tenside, Komplexbildner, Bleichmittel, Bleichaktivatoren, Färbund Duftstoffe, Antioxidantien, Gerüststoffe, Enzyme, Enzym-Stabilisatoren, antimikrobielle Wirkstoffe, Vergrauungsinhibitoren, Antiredepositionsmittel, pH-Stellmittel, Elektrolyte, Schauminhibitoren und UV-Absorber befinden.

Die Mikrokapseln können ferner kationische Tenside, Vitamine, Proteine, Konservierungsmittel, Waschkraftverstärker oder Perlglanzgeber enthalten. Die Füllungen der Mikrokapseln können Feststoffe oder Flüssigkeiten in Form von Lösungen oder Emulsionen bzw. Suspensionen sein.

Die Mikrokapseln können im herstellungsbedingten Rahmen eine beliebige Form aufweisen, sie sind jedoch bevorzugt näherungsweise kugelförmig. Ihr Durchmesser entlang ihrer größten räumlichen Ausdehnung kann je nach den in ihrem Inneren enthaltenen Komponenten und der Anwendung zwischen 0,01 µm (visuell nicht als Kapsel erkennbar) und 10.000 µm liegen. Bevorzugt sind sichtbare Mikrokapseln mit einem Durchmesser im Bereich von 100 µm bis 7.000 µm, insbesondere von 400 µm bis 5.000 µm. Die Mikrokapseln sind nach bekannten Verfahren zugänglich, wobei der Koazervation und der Grenzflächenpolymerisation die größte Bedeutung zukommt. Als Mikrokapseln lassen sich sämtliche auf dem Markt angebotenen tensidstabilen Mikrokapseln einsetzen, beispielsweise die Handelsprodukte (in Klammern angegeben ist jeweils das Hüllmaterial) Hallcrest Microcapsules (Gelatine, Gummi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose); Unicerin C30 (Lactose, mikrokristalline Cellulose, Hydroxypropylmethylcellulose), Kobo Glycospheres (modifizierte Stärke, Fettsäureester, Phospholipide), Softspheres (modifiziertes Agar Agar) und Kuhs Probiol Nanospheres (Phospholipide).

Alternativ können auch Partikel eingesetzt werden, die keine Kern-Hülle-Struktur aufweisen, sondern in denen der Wirkstoff in einer Matrix aus einem matrix-bildenden Material verteilt ist. Solche Partikel werden auch als "Speckies" bezeichnet.

Ein bevorzugtes matrix-bildendes Material ist Alginat. Zur Herstellung Alginat-basierter Speckies wird eine wässrige Alginat-Lösung, welche auch den einzuschließenden Wirkstoff bzw. die einzuschließenden Wirkstoffe enthält, vertropft und anschließend in einem Ca²⁺-Ionen oder Al³⁺-Ionen enthaltendem Fällbad ausgehärtet.

Alternativ können anstelle von Alginat andere, matrix-bildende Materialien eingesetzt werden. Beispiele für matrix-bildende Materialien umfassen Polyethylenglykol, Polyvinylpyrrolidon, Polymethacrylat, Polylysin, Poloxamer, Polyvinylalkohol, Polyacrylsäure, Polyethylenoxid, Polyethoxyoxazolin, Albumin, Gelatine, Acacia, Chitosan, Cellulose, Dextran, Ficoll®, Stärke, Hydroxyethylcellulose, Hydroxypropylcellulose, Hydroxypropylmethylcellulose, Hyaluronsäure, Carboxymethylcellulose, Carboxymethylcellulose, deacetyliertes Chitosan, Dextransulfat und Derivate dieser Materialien. Die Matrixbildung erfolgt bei diesen Materialien beispielsweise über Gelierung, Polyanion-Polykation-Wechselwirkungen oder Polyelektrolyt-MetallionWechselwirkungen. Die Herstellung von Partikeln mit diesen matrixbildenden Materialien ist an sich bekannt.

Die Partikel können stabil in den wässrigen flüssigen Wasch- oder Reinigungsmittel dispergiert werden. Stabil bedeutet, dass die Mittel bei Raumtemperatur und bei 40 °C über einen Zeitraum von mindestens 4 Wochen und bevorzugt von mindestens 6 Wochen stabil sind, ohne dass die Mittel aufrahmen oder sedimentieren. Die erfindungsgemäßen Verdicker bewirken durch die Viskositätserhöhung eine kinetische Verlangsamung der Sedimentation der Partikel und somit deren Stabilisierung im Schwebezustand.

Die Freisetzung der Wirkstoffe aus den Mikrokapseln oder Speckies erfolgt üblicherweise während der Anwendung der sie enthaltenden Mittel durch Zerstörung der Hülle bzw. der Matrix infolge mechanischer, thermischer, chemischer oder enzymatischer Einwirkung.

Die erfindungsgemäßen Wasch- oder Reinigungsmittel können zum Reinigen von textilen Flächengeweben und/oder harten Oberflächen verwendet werden. Erfindungsgemäße Reinigungsmittel können in Form eines Hand- oder Maschinengeschirrspülmittels, Allzweckreiniger für nicht-textile Oberflächen, z.B. aus Metall, lackiertem Holz oder Kunststoff, oder Reinigungsmittel für keramische Erzeugnisse, wie Porzellan, Fliesen, Kacheln vorliegen. Die Wasch- oder Reinigungsmittel können flüssig oder pastös formuliert werden.

Zur Herstellung der flüssigen Wasch- oder Reinigungsmittel können die Tenside, der Verdicker und die fakultativen Komponenten in beliebiger Reihenfolge miteinander vereint werden. Zum Beispiel können die sauren Komponenten wie beispielsweise die linearen Alkylsulfonate, Zitronensäure, Borsäure, Phosphonsäure, die Fettalkoholethersulfate, usw. vorgelegt und die nichtionischen Tenside zugegeben werden. Anschließend wird eine Base wie beispielsweise NaOH, KOH, Triethanolamin oder Monoethanolamin gefolgt von der Fettsäure, falls vorhanden, zugegeben. Darauffolgend werden die restlichen Inhaltsstoffe und die Lösungsmittel des wässrigen flüssigen Wasch- oder Reinigungsmittel zu der Mischung gegeben. Dann wird der erfindungsgemäße Verdicker zugegeben und, gegebenenfalls der pH-Wert korrigiert, z. B. auf einen Wert von 8 bis 9,5.

Gegebenenfalls können abschließend zu dispergierende Partikel zugegeben und durch Mischen homogen in dem wässrigen flüssigen Wasch- oder Reinigungsmittel verteilt werden.

Die Erfindung wird durch die folgenden Beispiele näher veranschaulicht.

### Vergleichsbeispiel 1: Herstellung eines Verdickers im einstufigen Emulsionspolymerisationsprozess

In einer Rührapparatur, bestehend aus einem 4 Liter HWS Gefäß mit Ankerrührer (150 Upm), Rückflusskühler, Innenthermofühler und Dosierstation, wurden als Vorlage 478,73 g entsalztes Wasser (VE-Wasser) und 10,71 g Emulgator Texapon NSO (Natriumlaurylethersulfat; 28%ig in Wasser) gemischt.

Dieser Lösung wurden bei 75 °C 16,29 g einer 7%igen wässrigen Natriumperoxodisulfat-Lösung zugegeben und die Mischung bei 75 °C 5 Minuten gerührt. Danach wurden bei 75 °C unter weiterem Rühren die Emulsion bestehend aus 714 g vollständig entsalztes Wasser (VE-Wasser), den Monomeren (183,67 g Methacrylsäure und 420 g Ethylacrylat) und dem Emulgator (21,43 g Texapon NSO-28%ig in Wasser) während 2 Stunden gleichmäßig zudosiert. Anschließend wurde die Reaktionsmischung noch 1 Stunde. bei 75°C gerührt und danach auf Raumtemperatur gebracht. Bei Raumtemperatur wurden 0,3 g einer 4 %igen Dissolvine® E-FE-6 Lösung (Eisen-II-salzlösung) und 12 g einer 5%igen Wasserstoffperoxidlösung zugegeben und 90 g einer 1 %igen Ascorbinsäure-Lösung während 30 Minuten gleichmäßig zudosiert.

### Vergleichsbeispiel 2: Herstellung eines Assoziativerdickers im einstufigen Emulsionspolymerisationsprozess

In einer Rührapparatur, bestehend aus einem 4 Liter HWS Gefäß mit Ankerrührer (150 Upm), Rückflusskühler, Innenthermofühler und Dosierstation, wurden als Vorlage 736,73 g entsalztes Wasser (VE-Wasser) und 10,71 g Emulgator Texapon NSO (28%ig in Wasser) gemischt.

Dieser Lösung wurden bei 75 °C 4,23 g einer 7%igen wässrigen Natriumperoxodisulfat-Lösung zugegeben und die Mischung bei 75 °C während 5 Minuten gerührt. Danach wurden bei 75 °C unter weiterem Rühren eine Emulsion bestehend aus 457,04 g vollständig entsalztes Wasser (VE-Wasser), den Monomeren (183,67 g Methacrylsäure und 360 g Ethylacrylat, 15 g Lutensol® AT 25 Methacrylat [= (C₁₆-₁₈)-(EO)₂₅-Methacrylat], 45 g Methylmethacrylat), und 10,71 g Emulgator Texapon NSO-28%ig in Wasser während 2 Stunden sowie gleichzeitig 12,06 g 7 %ige wässrige Natriumperoxodisulfat-Lösung während 2 Stunden gleichmäßig zudosiert. Anschließend wurde die Reaktionsmischung noch 1 Stunde bei 75°C gerührt und danach auf Raumtemperatur gebracht. Bei Raumtemperatur wurden 0,3 g einer 4 %igen Dissolvine® E-FE-6 Lösung (Eisen-II-salzlösung) und 12 g einer 5%igen Wasserstoffperoxidlösung zugegeben und 90 g einer 1 %igen Ascorbinsäure-Lösung während 30 Minuten gleichmäßig zudosiert.

### Vergleichsbeispiel 3: Herstellung eines Assoziativerdickers im einstufigen Emulsionspolymerisationsprozess

In einer Rührapparatur, bestehend aus einem 4 Liter HWS Gefäß mit Ankerrührer (150 Upm), Rückflusskühler, Innenthermofühler und Dosierstation, wurden als Vorlage 736,73 g entsalztes Wasser (VE-Wasser) und 10,71 g Emulgator Texapon NSO-28%ig in Wasser gemischt.

Dieser Lösung wurden bei 75 °C 4,23 g einer 7%igen wässrigen Natriumperoxodisulfat-Lösung zugegeben und die Mischung bei 75 °C während 5 Minuten gerührt. Danach wurden bei 75 °C unter weiterem Rühren die Emulsion bestehend aus 457,04 g vollständig entsalztes Wasser (VE-Wasser), den Monomeren (183,67 g Methacrylsäure und 300 g Ethylacrylat, 60 g n-Butylacrylat, 15 g Lutensol® AT 25 Methacrylat [= (C₁₆-₁₈)-(EO)₂₅-Methacrylat], 45 g Methylmethacrylat), 10,71 g Emulgator Texapon NSO-28%ig in Wasser während 2 Stunden sowie gleichzeitig 12,06 g 7 %ige wässrigen Natriumperoxodisulfat-Lösung während 2 Stunden gleichmäßig zudosiert (Emulsion und Natriumperoxodisulfat-Zulauf kombinieren). Anschließend wurde die Reaktionsmischung noch 1 Stunde bei 75°C gerührt und danach auf Raumtemperatur gebracht. Bei Raumtemperatur wurden 0,3 g einer 4 %igen Dissolvine® E-FE-6 Lösung (Eisen-II-salzlösung) und 12 g einer 5%igen Wasserstoffperoxidlösung zugegeben und 90 g einer 1 %igen Ascorbinsäure-Lösung während 30 Minuten gleichmäßig zudosiert.

### Beispiel 1: Erfindungsgemäße Herstellung der Assoziativverdicker-Dispersion mit Redox/Teilbatch-Fahrweise

In einer Rührapparatur, bestehend aus einem 2 Liter HWS Gefäß mit Ankerrührer (175 Upm), Rückflusskühler, Innenthermofühler und Dosierstation, wurden als Vorlage 540,17 g entsalztes Wasser (VE-Wasser) und 8,21 g Emulgator Texapon NSO-28%ig in Wasser gemischt.

Dieser Lösung wurden bei 75 °C 12,49 g einer 7%igen wässrigen Natriumperoxodisulfat-Lösung zugegeben und die Mischung bei 75 °C während 5 Minuten gerührt. Danach wurden bei 75 °C unter weiterem Rühren 50% der Emulsion bestehend aus 429,91 g vollständig entsalztes Wasser (VE-Wasser), den Monomeren (140,82 g Methacrylsäure, 138,0 g Ethylacrylat, 138,0 g n-Butylacrylat, 11,5 g Lutensol® AT 25 Methacrylat [= (C₁₆-₁₈)-(EO)₂₅- Methacrylat], 34,5 g Methylmethacrylat), und 16,73 g Emulgator Texapon NSO-28%ig in Wasser während 1 Stunde zudosiert. Anschließend wurde die Reaktionsmischung noch 1 Stunde bei 75°C gerührt. Nach dem Auspolymerisieren wurde die Restmenge (50%) der Emulsion zugegeben und 1 Stunde bei 60°C nachgerührt. Dann wurden 0,23 g einer 4 %igen Dissolvine® E-FE-6 Lösung (Eisen-II-salzlösung) und 9,2 g einer 5%igen Wasserstoffperoxidlösung zugegeben. 11,5 g einer 1 %igen Ascorbinsäure-Lösung wurden während 2 Stunden gleichmäßig zudosiert. Anschließend wird auf Raumtemperatur gekühlt.

Die übrigen in den nachstehenden Tabelle 1 b bis 1 d aufgeführten erfindungsgemäßen Dispersionen, Beispiel 2 bis Beispiel 21, wurden analog hergestellt. Die Mengenangaben für die Einsatzstoffe sind in Teilen pro 100 reaktive Monomerteile (parts per hundred monomers; pphm) angegeben. Bei der Charakterisierung der Dispersion wurden folgende Werte gemessen:

Feststoffgehalt: Die Dispersion wurde 30 min bei 140 °C getrocknet und der Feststoffgehalt in Prozent aus dem Verhältnis Trockenrückstand zu Einwaage bestimmt.

Teilchengröße: Die Dispersion wurde auf 0,01 % verdünnt und die Teilchengröße über Lichtstreuung im High Performance Particle Sizer 5001 (HPPS) von der Firma Malvern Instruments gemessen.

LD-Wert: Die Dispersion wurde auf 0,01 % verdünnt und die Lichtdurchlässigkeit (LD) der Dispersion im Vergleich zu reinem Wasser als ein Maß für die Teilchengröße optisch im Hach DR/2010 gemessen.

| | |
|---|---|
| PLEX® 6877-O in MMA | Methacrylsäureester eines ethoxylierten (25 mol EO) C₁₆-C₁₈-Fettalkoholgemisches 25 %ig in Methylmethacrylat |
| NaPS | Natriumperoxidisulfat |
| n.b. | nicht bestimmt |

**Tabelle 1a:**

| Verdicker-Dispersion | Vergleich 1 | Vergleich 2 | Vergleich 3 |
|---|---|---|---|
| n-Butylacrylat (pphm) | -- | -- | 10 |
| Ethylacrylat (pphm) | 70 | 60 | 50 |
| Methacrylsäure (pphm) | 30 | 30 | 30 |
| PLEX® 6877-O 25%ig in MMA (pphm) | -- | 10 | 10 |
| Texapon NSO Vorlage/E-Zulauf (pphm) | 0,5/1 | 0,5/0,5 | 0,5/0,5 |
| NaPS in Vorlage (pphm) | 0,19 | 0,05 | 0,05 |
| NaPS - Zulauf (pphm) | -- | 0,14 | 0,14 |
| Polymerisationstemp. (°C) | 75 | 75 | 75 |
| Feststoffgehalt (%) | 30,5 | 30,2 | 30,4 |
| Teilchengröße (nm) | 74 | 66 | 62 |
| LD - 0,01%ig (%) | 97 | 98 | 98 |

**Tabelle 1b: Erfindungsgemäß hergestellte Verdickerdispersionen, polymerisiert mit Quellung**

| Verdicker-Dispersion | Beispiel 1 | Beispiel 2 | Beispiel 3 | Beispiel 4 | Beispiel 5 | Beispiel 6 | Beispiel 7 |
|---|---|---|---|---|---|---|---|
| n-Butylacrylat (pphm) | 30 | 30 | 30 | 30 | 30 | 30 | 30 |
| Ethylacrylat (pphm) | 30 | 30 | 30 | 30 | 30 | 30 | 30 |
| Methacrylsäure (pphm) | 30 | 30 | 30 | 30 | 30 | 30 | 30 |
| PLEX 6877-O 25%ig in MMA (pphm) | 10 | 10 | 10 | 10 | 10 | 10 | 10 |
| Texapon NSO Vorlage/E-Zulauf (pphm) | 0,5/1 | 0,5/1 | 0,5/1 | 0,5/1 | 0,5/1 | 0,5/1 | 0,5/1 |
| NaPS in Vorlage (pphm) | 0,19 | 0,19 | 0,19 | 0,19 | 0,19 | 0,19 | 0,19 |
| NaPS - Zulauf (pphm) | -- | -- | -- | -- | -- | -- | -- |
| NaPS-Zugabe (pphm) | -- | -- | -- | -- | -- | -- | -- |
| Wasserstoffperoxid-Zugabe (pphm) | 0,1 | 0,1 | 0,1 | 0,1 | 0,1 | 0,1 | 0,1 |
| Ascorbinsäure (pphm) | 0,025 | 0,015 | 0,15 | 0,15 | 0,15 | 0,15 | 0,1 |
| Polymerisationstemp. (°C) | 75 | 75 | 75 | 75 | 75 | 75 | 75 |
| Zugabe der restl. Monomer-emulsion (min.) | 60 | 5 | 5 | 5 | 5 | 5 | 5 |
| Quellung (min) | 60 | etwa 55 | etwa 45 | etwa 35 | etwa 30 | etwa 25 | etwa 45 |
| Teilbatchausgangstemp. (°C) | 60 | 23 | 30 | 40 | 50 | 60 | 30 |
| Feststoffgehalt (%) | 30,6 | 30,6 | 30,5 | 30,3 | 30,5 | 30,7 | 30,6 |
| Teilchengröße (nm) | 62 | 64 | 64 | 66 | 64 | 64 | 63 |
| LD - 0,01%ig (%) | 98 | 98 | 98 | 98 | 98 | 98 | 98 |

**Tabelle 1 c: Erfindungsgemäß hergestellte Verdickerdispersionen, polymerisiert mit Quellung.**

| Verdicker-Dispersion | Beispiel 8 | Beispiel 9 | Beispiel 10 | Beispiel 11 | Beispiel 12 | Beispiel 13 | Beispiel 14 |
|---|---|---|---|---|---|---|---|
| n-Butylacrylat (pphm) | 30 | 30 | 30 | 30 | | 60 | 35 |
| Ethylacrylat (pphm) | 30 | 30 | 30 | 30 | 60 | | 35 |
| Methacrylsäure (pphm) | 30 | 30 | 30 | 30 | 30 | 30 | 30 |
| Acrylsäure (pphm) | -- | -- | -- | -- | -- | -- | -- |
| PLEX 6877-O 25%ig in MMA (pphm) | 10 | 10 | 10 | 10 | 10 | 10 | -- |
| Texapon NSO Vorlage/E-Zulauf (pphm) | 0,5/1 | 0,5/2 | 0,5/1 | 0,5/1 | 0,5/1 | 0,5/1 | 0,5/1 |
| NaPS in Vorlage (pphm) | 0,19 | 0,19 | 0,19 | 0,19 | 0,19 | 0,19 | 0,19 |
| NaPS - Zulauf (pphm) | -- | -- | -- | -- | -- | -- | -- |
| NaPS-Zugabe (pphm) | -- | 0,1 | - | 0,1 | 0,1 | 0,1 | 0,1 |
| Wasserstoffperoxid-Zugabe (pphm) | 0,1 | -- | 0,1 | -- | -- | -- | -- |
| Ascorbinsäure (pphm) | 0,05 | 0,15 | 0,025 | 0,15 | 0,15 | 0,15 | 0,15 |
| Polymerisationstemp. (°C) | 75 | 75 | 75 | 75 | 75 | 75 | 75 |
| Zugabe der restl. Monomer-emulsion (min.) | 5 | 5 | 5 | 5 | 60 | 60 | 60 |
| Quellung (min.) | 60 | 60 | 60 | 60 | 60 | 60 | 60 |
| Teilbatchausgangstemp. (°C) | 60 | 60 | 60 | 50 | 60 | 60 | 60 |
| Feststoffgehalt (%) | 30,3 | 30,6 | 30,6 | 29,5 | 30,5 | 29,8 | 30,1 |
| Teilchengröße (nm) | 55 | 67 | 62 | 66 | 77 | 74 | 66 |
| LD - 0,01%ig (%) | 98 | 98 | 98 | 97 | 98 | 96 | 97 |

**Tabelle 1d Erfindungsgemäße Verdickerdispersionen, redox/teilbatch polymerisiert mit Quellung.**

| Verdicker-Dispersion | Beispiel 15 | Beispiel 16 | Beispiel 17 | Beispiel 18 | Beispiel 19 | Beispiel 20 | Beispiel 21 |
|---|---|---|---|---|---|---|---|
| n-Butylacrylat (pphm) | -- | 70 | 30 | 50 | 30 | 60 | 30 |
| Ethylacrylat (pphm) | 70 | -- | 20 | -- | 30 | -- | 20 |
| Methacrylsäure (pphm) | 30 | 30 | 40 | 40 | 40 | 40 | 30 |
| Acrylsäure (pphm) | -- | -- | -- | -- | -- | -- | 10 |
| PLEX 6877-O 25%ig in MMA (pphm) | -- | -- | 10 | 10 | -- | -- | 10 |
| Texapon NSO Vorlage/E-Zulauf (pphm) | 0,5/1 | 0,5/1 | 0,5/1 | 0,5/1 | 0,5/1 | 0,5/1 | 0,5/1 |
| NaPS in Vorlage (pphm) | 0,19 | 0,19 | 0,19 | 0,19 | 0,19 | 0,19 | 0,19 |
| NaPS - Zulauf (pphm) | | | | | | | |
| NaPS-Zugabe (pphm) | 0,1 | 0,1 | 0,1 | 0,1 | 0,1 | 0,1 | 0,1 |
| Wasserstoffperoxid-Zugabe (pphm) | -- | -- | -- | -- | -- | -- | -- |
| Ascorbinsäure (pphm) | 0,15 | 0,15 | 0,15 | 0,15 | 0,15 | 0,15 | 0,15 |
| Polymerisationstemp. (°C) | 75 | 75 | 75 | 75 | 75 | 75 | 75 |
| Zugabe der restl. Monomer-emulsion (min.) | 60 | 60 | 60 | 60 | 60 | 60 | 60 |
| Quellung (min.) | 60 | 60 | 60 | 60 | 60 | 60 | 60 |
| Teilbatchausgangstemp. (°C) | 60 | 60 | 60 | 60 | 60 | 60 | 60 |
| Feststoffgehalt (%) | 30,0 | 30,2 | 30,5 | 29,3 | 30,2 | 28,6 | 30,2 |
| Teilchengröße (nm) | 73 | 64 | 74 | 75 | 72 | 64 | 87 |
| LD - 0,01%ig (%) | 97 | 98 | 96 | 96 | 97 | ? | ? |

### Herstellung eines Flüssigwaschmittels

Man stellte die folgenden Stammformulierungen her (Gew.-%, bezogen auf die Fertigformulierung):

| | Formulierung 1 | Formulierung 2 |
|---|---|---|
| Lutensit® A-LBS (99%; lineares Alkylsulfonat in Säureform) | 17,92 | 13,44 |
| Lutensol® AO 7 (nichtionische Tensid) | 20 | 10 |
| Kokosfettsäure Edenor K8-18 | 8,5 | 8,5 |
| KOH | 5 | 4,38 |
| Natriumcitrat-dihydrat | 3 | 3 |
| 1,2-Propylenglykol | 8 | 8 |
| Ethanol | 2 | 2 |
| Wasser | qs | qs |

Die obigen Bestandteile wurden gemischt und mit Wasser auf 90 Gew.-% aufgefüllt, d.h. es verblieb eine Formulierungslücke von 10 Gew.-%. Die Stammformulierungen wurden mit KOH auf pH 8,6 eingestellt.

Für die (unverdickten) Referenzformulierungen wurden die Stammformulierungen mit Wasser auf 100 Gew.-% aufgefüllt. Für die verdickten Testformulierungen wurden die Stammformulierungen mit Verdickerdispersion und Wasser aufgefüllt, so dass sich unter Berücksichtigung des Feststoffgehalts der Dispersion eine Verdickerkonzentration von 1,5 Gew.-%, bezogen auf die Fertigformulierung, einstellte. Vor der Viskositätsmessung ließ man die Formulierungen wenigstens 5 Stunden ruhen.

### Bestimmung der Low-shear-Viskosität

Unter Berücksichtigung der Vorschriften nach DIN 51550, DIN 53018, DIN 53019 wurden mit dem Brookfield Viskosimeter Modell RV-03 bei einer Drehzahl von 20 Umdrehungen pro Minute mit der Spindel Nr. 62 Viskositäten von etwa 1000 mPas gemessen.

Die Transmission wurde in % bei 440 nm bei 23 °C gemessen. Die gefundenen Werte sind als Prozentwert, relativ zur Transmission der unverdickten Referenzformulierung angegeben.

Die Ergebnisse sind in den Tabellen 2 und 3 zusammengefasst.

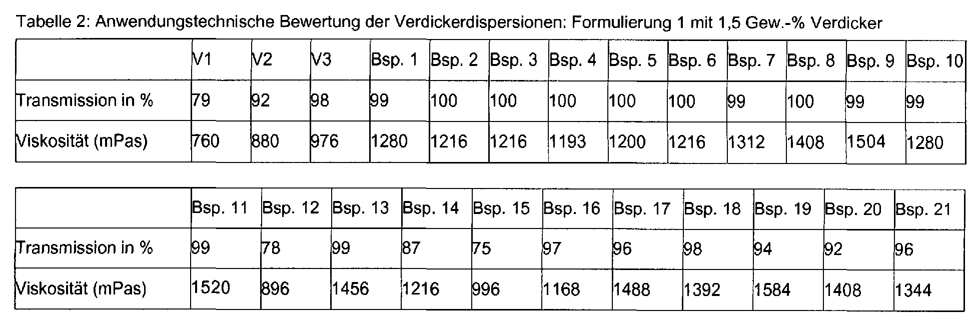

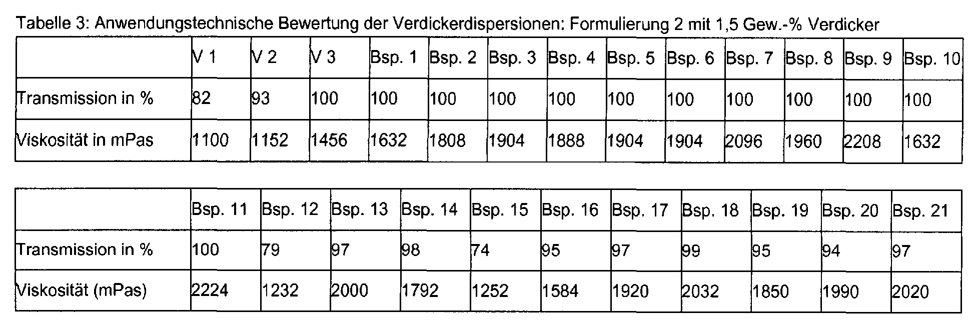

## Patentansprüche

1. Verfahren zur Herstellung einer wässrigen Verdicker-Dispersion aus einer Monomerzusammensetzung, die umfasst:
a) wenigstens eine ethylenisch ungesättigten Carbonsäure, und
b) wenigstens ein ethylenisch ungesättigtes hydrophobes Monomer,
wobei man
(i) aus 10 bis 80 Gew.-% der Monomerzusammensetzung eine zumindest teilweise polymerisierte Voremulsion herstellt und
(ii) die Restmenge der Monomerzusammensetzung vollständig zu der zumindest teilweise polymerisierten Voremulsion zugibt und mittels eines Redoxinitiatorsystems eine radikalische Polymerisation initiiert.

2. Verfahren nach Anspruch 1, wobei man im Schritt (i) die zumindest teilweise polymerisierte Voremulsion in Gegenwart eines thermisch aktivierbaren Initiators oder eines Redoxinitiators herstellt.

3. Verfahren nach Anspruch 1 oder 2, wobei man im Schritt (i) die zumindest teilweise polymerisierte Voremulsion durch Monomer-Zulauffahrweise herstellt.

4. Verfahren nach Anspruch 3, wobei man im Schritt (i) den Initiator im Wesentlichen vollständig vorlegt.

5. Verfahren nach einem der vorhergehenden Ansprüche, wobei man wobei man im Schritt (ii) die radikalische Polymerisation durch Zugabe einer Reduktionsmittelkomponente eines Redoxinitiatorsystems initiiert, wobei man zuvor eine Oxidationsmittelkomponente eines Redoxinitiatorsystems zugegeben hat und/oder überschüssiger Initiator aus Schritt (i) als eine Oxidationsmittelkomponente eines Redoxinitiatorsystems fungiert.

6. Verfahren nach einem der Ansprüche 1 bis 4, wobei man im Schritt (ii) die radikalische Polymerisation durch im Wesentlichen gleichzeitige Zugabe einer Oxidationsmittelkomponente eines Redoxinitiators und einer Reduktionsmittelkomponente eines Redoxinitiators initiiert.

7. Verfahren nach einem der vorhergehenden Ansprüche, wobei man im Schritt (ii) die zumindest teilweise polymerisierten Voremulsion in Gegenwart der Restmenge der Monomerzusammensetzung wenigstens eine Stunde quellen lässt, bevor man die radikalische Polymerisation initiiert.

8. Verfahren nach einem der Ansprüche 1 bis 6, wobei man im Schritt (ii) die Restmenge der Monomerzusammensetzung innerhalb einer Zeitspanne von weniger als einer Stunde zu der zumindest teilweise polymerisierten Voremulsion zugibt und nach vollständiger Zugabe die radikalische Polymerisation unverzüglich initiiert.

9. Verfahren nach einem der vorhergehenden Ansprüche, wobei das ethylenisch ungesättigte hydrophobe Monomer ausgewählt ist unter C₁-C₉-Alkyl(meth)-acrylaten, Dienen, Vinylaromaten, (Meth)acrylnitril und Gemischen davon.

10. Verfahren nach einem der vorhergehenden Ansprüche, wobei die Monomerzusammensetzung außerdem wenigstens ein ethylenisch ungesättigtes Assoziativmonomer umfasst.

11. Verfahren nach Anspruch 10, wobei das ethylenisch ungesättigte Assoziativmonomer ausgewählt ist unter C₁₀-C₃₀-Alkyl(meth)acrylaten und ethylenisch ungesättigten Tensidmonomeren.

12. Verfahren nach Anspruch 11, wobei das ethylenisch ungesättigte Tensidmonomer die allgemeine Formel aufweist
R-O-(CH₂-CHR'-O)ₙ-CO-CR"=CH₂
worin R für C₆-C₃₀-Alkyl steht,
R' für Wasserstoff oder Methyl steht,
R" für Wasserstoff oder Methyl steht, und
n für eine Zahl von 2 bis 100 steht.

13. Verfahren nach einem der vorhergehenden Ansprüche, wobei die ethylenisch ungesättigte Carbonsäure ausgewählt ist unter Acrylsäure, Methacrylsäure, Itaconsäure und Maleinsäure.

14. Verfahren nach einem der vorhergehenden Ansprüche, wobei die Herstellung der zumindest teilweise polymerisierten Voremulsion und/oder die Polymerisation der Restmenge der Monomerzusammensetzung in Gegenwart eines anionischen und/oder nichtionischen Emulgators erfolgt.

15. Verdicker-Dispersion, erhältlich durch ein Verfahren nach einem der vorhergehenden Ansprüche.

## Claims

1. A method of preparing an aqueous thickener dispersion from a monomer composition which comprises:
a) at least one ethylenically unsaturated carboxylic acid, and
b) at least one ethylenically unsaturated hydrophobic monomer,
where
(i) an at least partially polymerized pre-emulsion is prepared from 10 to 80% by weight of the monomer composition and
(ii) the remainder of the monomer composition is added in its entirety to the at least partially polymerized pre-emulsion and a free-radical polymerization is initiated by means of a redox initiator system.

2. The method according to claim 1, where, in step (i), the at least partially polymerized pre-emulsion is prepared in the presence of a thermally activatable initiator or a redox initiator.

3. The method according to claim 1 or 2, where, in step (i), the at least partially polymerized pre-emulsion is prepared by monomer feed procedure.

4. The method according to claim 3, where, in step (i), the initiator is essentially initially introduced in its entirety.

5. The method according to any one of the preceding claims, where, in step (ii), the free-radical polymerization is initiated by adding a reducing agent component of a redox initiator system, where an oxidizing agent component of a redox initiator system has been added beforehand and/or excess initiator from step (i) acts as an oxidizing agent component of a redox initiator system.

6. The method according to any one of claims 1 to 4, where, in step (ii), the free-radical polymerization is initiated through essentially simultaneous addition of an oxidizing agent component of a redox initiator and of a reducing agent component of a redox initiator.

7. The method according to any one of the preceding claims, where, in step (ii), the at least partially polymerized pre-emulsion is left to swell in the presence of the remainder of the monomer composition for at least one hour before the free-radical polymerization is initiated.

8. The method according to any one of claims 1 to 6, where, in step (ii), the remainder of the monomer composition is added to the at least partially polymerized pre-emulsion within a period of time of less than one hour and, following complete addition, the free-radical polymerization is initiated immediately.

9. The method according to any one of the preceding claims, where the ethylenically unsaturated hydrophobic monomer is selected from C₁-C₉-alkyl (meth)acrylates, dienes, vinyl aromatics, (meth)acrylonitrile and mixtures thereof.

10. The method according to any one of the preceding claims, where the monomer composition furthermore comprises at least one ethylenically unsaturated associative monomer.

11. The method according to claim 10, where the ethylenically unsaturated associative monomer is selected from C₁₀-C₃₀-alkyl (meth)acrylates and ethylenically unsaturated surfactant monomers.

12. The method according to claim 11, where the ethylenically unsaturated surfactant monomer has the general formula
R-O-(CH₂-CHR'-O)ₙ-CO-CR"=CH₂
in which R is C₆-C₃₀-alkyl,
R' is hydrogen or methyl,
R" is hydrogen or methyl, and
n is a number from 2 to 100.

13. The method according to any one of the preceding claims, where the ethylenically unsaturated carboxylic acid is selected from acrylic acid, methacrylic acid, itaconic acid and maleic acid.

14. The method according to any one of the preceding claims, where the preparation of the at least partially polymerized pre-emulsion and/or the polymerization of the remainder of the monomer composition takes place in the presence of an anionic and/or nonionic emulsifier.

15. A thickener dispersion obtainable by a method according to any one of the preceding claims.

## Revendications

1. Procédé de fabrication d'une dispersion aqueuse d'épaississant à partir d'une composition de monomères, qui comprend :
a) au moins un acide carboxylique éthyléniquement insaturé et
b) au moins un monomère hydrophobe éthyléniquement insaturé,
selon lequel
(i) une pré-émulsion au moins partiellement polymérisée est fabriquée à partir de 10 à 80 % en poids de la composition de monomères et
(ii) la quantité restante de la composition de monomères est ajoutée en totalité à la pré-émulsion au moins partiellement polymérisée et une polymérisation radicalaire est initiée par un système initiateur redox.

2. Procédé selon la revendication 1, dans lequel, à l'étape (i), la pré-émulsion au moins partiellement polymérisée est fabriquée en présence d'un initiateur thermiquement activable ou d'un initiateur redox.

3. Procédé selon la revendication 1 ou 2, dans lequel, à l'étape (i), la pré-émulsion au moins partiellement polymérisée est fabriquée par un mode d'alimentation des monomères.

4. Procédé selon la revendication 3, dans lequel l'initiateur est chargé initialement essentiellement en totalité lors de l'étape (i).

5. Procédé selon l'une quelconque des revendications précédentes, dans lequel, à l'étape (ii), la polymérisation radicalaire est initiée par ajout d'un composant réducteur d'un système initiateur redox, un composant oxydant d'un système initiateur redox ayant été ajouté auparavant et/ou l'initiateur en excès de l'étape (i) jouant le rôle de composant oxydant d'un système initiateur redox.

6. Procédé selon l'une quelconque des revendications 1 à 4, dans lequel, à l'étape (ii), la polymérisation radicalaire est initiée par ajout essentiellement simultané d'un composant oxydant d'un initiateur redox et d'un composant réducteur d'un initiateur redox.

7. Procédé selon l'une quelconque des revendications précédentes, dans lequel, à l'étape (ii), la pré-émulsion au moins partiellement polymérisée est laissée gonfler pendant au moins une heure en présence de la quantité restante de la composition de monomères, avant d'initier la polymérisation radicalaire.

8. Procédé selon l'une quelconque des revendications 1 à 6, dans lequel, à l'étape (ii), la quantité restante de la composition de monomères est ajoutée en une durée inférieure à une heure à la pré-émulsion au moins partiellement polymérisée et la polymérisation radicalaire est initiée immédiatement après l'ajout complet.

9. Procédé selon l'une quelconque des revendications précédentes, dans lequel le monomère hydrophobe éthyléniquement insaturé est choisi parmi les (méth)acrylates d'alkyle en C₁-C₉, les diènes, les composés aromatiques de vinyle, le (méth)acrylonitrile et leurs mélanges.

10. Procédé selon l'une quelconque des revendications précédentes, dans lequel la composition de monomères comprend également au moins un monomère associatif éthyléniquement insaturé.

11. Procédé selon la revendication 10, dans lequel le monomère associatif éthyléniquement insaturé est choisi parmi les (méth)acrylates d'alkyle en C₁₀-C₃₀ et les monomères tensioactifs éthyléniquement insaturés.

12. Procédé selon la revendication 11, dans lequel le monomère tensioactif éthyléniquement insaturé présente la formule générale
R-O-(CH₂-CHR'-O)ₙ-CO-CR''=CH₂
dans laquelle R représente un alkyle en C₆-C₃₀,
R' représente l'hydrogène ou un méthyle,
R" représente l'hydrogène ou un méthyle, et
n représente un nombre de 2 à 100.

13. Procédé selon l'une quelconque des revendications précédentes, dans lequel l'acide carboxylique éthyléniquement insaturé est choisi parmi l'acide acrylique, l'acide méthacrylique, l'acide itaconique et l'acide maléique.

14. Procédé selon l'une quelconque des revendications précédentes, dans lequel la fabrication de la pré-émulsion au moins partiellement polymérisée et/ou la polymérisation de la quantité restante de la composition de monomères ont lieu en présence d'un émulsifiant anionique et/ou non ionique.

15. Dispersion d'épaississant, pouvant être obtenue par un procédé selon l'une quelconque des revendications précédentes.
